# EUROPEAN PATENT APPLICATION

(11) **EP 2 319 394 A1**
(43) Date of publication of application: **11.05.2011**
(21) Application number: 09382240.1
(22) Date of filing: 05.11.2009
(51) Int. Cl.: A61B 5/00

(54) **Non-invasive device and method for monitoring analytes in biological samples**

(71) Applicant: Rus Medical Technology S.A., 37185 Villamayor de la Armuña, Salamanca (ES)
(72) Inventor: Ródenas Santiago, Juan Rafael, 28850, Torrejón de Ardoz (Madrid) (ES); Cano Ródenas, Valentín, 28850, Torrejón de Ardoz (Madrid) (ES)
(74) Representative: Elzaburu Marquez, Alberto

(57) **Abstract**

Device and method for determining analytes in biological samples (1) in a non-invasive way based on near infrared (NIR) spectroscopy through a sample or biological medium such as the finger of a hand. The patient's biological medium is placed in a receptacle (30,31) and the optical detector (23) receives the light originating from the source (25), which is attenuated by the biological medium. This measurement is corrected by the results of measurements of various physiological skin parameters obtained using a series of specific sensors, such as elasticity, hydration, pH, temperature or melanin levels. These sensors are connected to the glucose monitoring equipment which includes a mathematical algorithm correcting the light values in relation to the results of the measurements of the said physiological characteristics for each individual, thus producing a measurement of analytes in biological samples which is more accurate than the devices in the prior art.

## Description

### Scope of the invention

This invention relates to a non-invasive device for measuring analytes, preferably glucose, in a biological sample by near infrared spectroscopy (NIR), as well as a method for measuring an analyte in a biological sample using near infrared spectroscopy.

### Background of the invention

It is estimated that 170 million people throughout the world have diabetes, a disease in which the body does not produce insulin or respond adequately to it. A high concentration of glucose levels in blood (also known as blood sugar levels) can cause serious harm to the heart, blood vessels, kidneys, eyes and nerves. If untreated, diabetes can lead to unexpected death in a short period of time. Persons with diabetes need to balance their diet, exercise and medication (for example insulin, which can be taken orally or by injection), to maintain their blood glucose levels as close to normal levels as possible. Insulin pumps have been developed so that insulin can be administered to diabetic patients continuously.

Regardless of how insulin is administered to a diabetic, continuous monitoring of blood glucose level is very important to avoid the problems which arise when glucose levels are low or, conversely, excessively high. Thus diabetics have to monitor their blood glucose levels frequently (such as six times per day) to maintain an adequate level of insulin in the blood.

Many non-invasive systems and methods for measuring body biological parameters have been described in recent years, and these include non-invasive methods for the measurement of blood glucose. The following are some of these documents:

In US 4055768 entitled "Light measuring apparatus" by Nathan S. Bromberg, a fluorimeter is used to measure the concentration of a fluorescent material in a sample; an intermittent light to illuminate the sample with exciting radiation is used for this purpose. The intensity of the fluorescence emitted by the sample is modulated in a flash manner. The modulated fluorescence is converted into an electrical signal by a photodetector. The electrical signal is amplified and passes through a sensitive detector stage to an integrator. When the sample has been subjected to a particular quantity of excited radiation the signal marking the end of the integration period is generated. The signal accumulating in the integrator is then measured to obtain a measurement of the quantity of fluorescent material in the sample. The apparatus is also useful as a nephelometer for measuring the dispersion properties of light in a material illuminated by intermittent light.

In US 4704029 entitled "Blood glucose monitor" by Alan Van Heuvelen, a blood glucose monitor is used as an implant to control an insulin pump. The glucose monitor measures the level in blood using a refractometer which measures the refractive index of the blood at an interface with a transparent surface of the refractometer. The angle of incidence of the light is selected to be slightly less than the critical angle for total internal reflection, with the result that the reflected intensity varies enormously with the refractive index and the glucose concentration. A differential amplifier compares the intensity of the light reflected from the blood and the intensity of the beam prior to reflection. The output signal from the differential amplifier indicates only a change in the intensity of the reflected light caused by a change in the glucose concentration from a standard configuration.

In US 3963019 entitled "Ocular test method and apparatus" by Quandt, a beam of polarised light is directed across the aqueous humour of the patient's eye. An analyser detects the ray leaving the patient's eye and compares the effect which the aqueous humour has on that beam in relation to a reference beam. An excess or deficiency of glucose in the aqueous humour produces a positive or negative change in the output beam which indicates that the patient is in a condition of hyperglycaemia or hypoglycaemia.

In US 3958560 entitled "Automatic non-invasive glucose sensor" by Wayne Fort March, the patient's eye is automatically scanned using a source of radiation on one side of the cornea. The sensor located on the other side of the cornea detects the radiation passing through it. The glucose level in the patient's blood flow is a function of the quantity of radiation detected on the other side of the patient's cornea. The result is transmitted to a remote receiver which is coupled to a reading device which provides non-invasive glucose determinations.

In US 4014321 entitled "Non-invasive glucose sensor system" also by Wayne Fort March, the patient's eye is automatically scanned using a double source of polarised radiation, each of which transmits a different wavelength to one side of the patient's cornea. A sensor located on the other side of the cornea detects the optical rotation of the radiation passing through it. The glucose level in the patient's blood is a function of the amount of optical rotation in the radiation detected on the other side of the cornea. The result is transmitted to a receiver which is connected to a reading device providing non-invasive glucose determinations of a highly specific and reliable nature.

In US 4901728 entitled "Personal glucose monitor" by Donald P. Hutchinson, two polarisation states at right angles with equal minimum absorption of infrared light are transmitted through a tissue containing blood and a precise determination of the change in the intensity of the signal is obtained from the angle of rotation of those states. This rotation is a function of the glucose level. Another two polarisation states at right angles having the same wavelength of infrared light have to be selected to maximise absorption in order to compensate for absorption in the tissue.

In US 5028787 entitled "Non-invasive glucose measurement" by Robert D. Rosenthal, quantitative analysis equipment in the near infrared can be used for the non-invasive measurement of glucose in blood through analysing the zone of the spectrum corresponding to the near infrared, and shows the interactions between the energy and venous or arterial blood, or transmission through the blood present in part of the body.

In US 4882492 entitled "Non-invasive measurement of analyte concentrations in blood in the near infrared" by Kenneth J. Schlager, the apparatus and method used are based on dispersion correlation spectroscopy and applied to the analysis of blood in serum. The near infrared light is spectrally modified as it passes through the sample containing the analyte and is divided into two beams, one of which passes through a negative correlation filter which blocks out light in the absorption bands for the analyte being measured, while the other passes through a neutral filter capable of blocking light of all wavelengths in the range of interest in the same way. The difference in the intensity of the light between the two light paths provides a measurement which is proportional to the analyte concentration.

In the article by H. Zeller, et al. "Measurement of glucose in blood by infrared spectroscopy", p. 129 - 134, (1989), the glucose absorption spectra in blood and some other blood components are analysed with a view to identifying the wavelength ranges in which evidence of blood glucose can be detected. The differences between the absorption spectra for water alone and water plus glucose are observed in only one wavelength range, which is referred to as the "fingerprint region", extending from 1650 to 800 cm⁻¹ .

In the article by Yitzhak Mendelson et al., "Blood glucose measurement by multiple attenuation total reflection spectroscopy and absorption spectroscopy in the infrared" (IEEE Transactions on Biomedical Engineering, 17, 1990), it is noted that of the more than 20 absorption peaks for D-glucose in the 2.5-10 micron range, not all the peaks are specific to D-glucose. However, the peak at a wavelength of 640 cm⁻¹ is prominent and is attributed to the carbon-oxygen-carbon bonds in the pyran ring of the D-glucose. It is also commented that because of the high background absorption due to water in the wavelength range in the region of the spectrum corresponding to the infrared, and the relatively low concentration of glucose in blood, it is important to use a CO₂ laser because it produces a powerful beam with the appearance of narrow peaks, improving detection efficiency at these very low concentrations. Sensitivity is increased through the use of attenuated total multiple reflection (ATR) by passing the laser beam through the sample on various occasions. Unfortunately the application of this laser/ATR equipment is very expensive.

Because of their complicated structures biological molecules have many similar absorption peaks in the infrared, which are frequently superimposed. For example the characteristic infrared spectrum of anhydrous D-glucose (ADG) has more than 20 absorption peaks in the wavelength range of 2.5-10 microns. It is important to point out that not all the absorption peaks are specific to this molecule. The prominent absorption peak around 9.61 microns (1040 cm⁻¹) is however quite specific for the carbon-oxygen-carbon in glucose, because of its pyran ring.

### Summary of the invention

All the documents in the prior art nevertheless suffer from one disadvantage: that is, being in many cases measurements based on spectrophotometric methods in which the measurement is as a consequence produced by measuring the light dispersed, reflected or transmitted through the subject's skin, differences caused by the different physiological characteristics of the skin of every individual are not however taken into account. Thus for example the methods and devices in the prior art do not take into account the deviations in glucose measurement which are produced as a consequence of, for example, the individual's skin being black, white or of any other colour, or of whether it is hydrated to a greater or lesser extent, or also has a greater or lesser pH. All these physiological characteristics have an effect on glucose measurement, and if they are not taken into account the measurement will always have an intrinsically high degree of inaccuracy.

As a consequence, a first aspect of the invention relates to a device for the non-invasive monitoring of an analyte in a sample or biological medium (1) which comprises:
- at least one light emitter (25) which emits light radiation at a plurality of wavelengths selected in the near infrared (NIR),
- a receptacle for receiving the sample or biological medium (1) which is being monitored,
- at least one light receptor (23) positioned in such a way that it can receive the light radiation originating from the said light source (25) after passing through the said sample or biological medium (1) and which has been attenuated by the same, in which the said at least one light receptor (23) is capable of generating at least one output signal which is indicative of the intensity of the light radiation attenuated by the sample or biological medium (1), and
- a signal processor connected to the said light receptor (23) which is capable of receiving the at least one output signal emitted by the same and generating at least one signal indicative of the concentration of the analyte in the said sample or biological medium (1),
   characterised in that the device also comprises:
- a plurality of sensors (20, 21, 22) for physiological parameters of the sample or biological medium (1) which generate values for each of the said parameters which are specific for the sample or biological medium (1) which is being monitored,
- a linearisation module which processes the values generated by the plurality of sensors (20, 21, 22) for physiological parameters of the sample or biological medium (1) and which produces a linearisation algorithm from those values, and
- a module for determining an analyte in the sample or biological medium (1) which on the basis of the at least one signal indicative of the concentration of analyte in the sample or biological medium (1) generated by the signal processor and the linearisation algorithm generated by the linearisation module determines the concentration of analyte in the sample or biological medium (1) corrected by the linearisation algorithm.

In a second aspect the invention relates to a non-invasive method for measuring an analyte in the sample or biological medium (1) using near infrared spectroscopy (NIR) which comprises the stages of:
- illuminating the sample or biological medium (1) with light radiation at a plurality of wavelengths selected in the near infrared (NIR),
- detecting the said light after it has been attenuated by the sample or biological medium (1),
- generating at least one output signal indicative of the intensity of the light radiation attenuated by the sample or biological medium (1) from the said attenuated light, and
- generating at least one signal indicative of the concentration of the analyte in the sample or biological medium (1) on the basis of the at least one output signal indicative of the intensity of the light radiation attenuated by the sample or biological medium (1),
   characterised in that the method further comprises the stages of:
- measuring a plurality of physiological parameters in the sample or biological medium (1), generating values for each of the said physiological parameters which are specific to the sample or biological medium (1) which is being monitored,
- generating a linearisation algorithm from the values of each of the physiological parameters which are specific to the sample or biological medium (1) which is being monitored, and which produces a linearisation algorithm from those values, and
- determining the concentration of the analyte in the sample or biological medium (1) corrected by the linearisation algorithm from the at least one signal indicative of the concentration of the analyte in the sample or biological medium (1) and the linearisation algorithm.

The devices and methods according to the invention are therefore capable of providing a measurement of the analyte in question, preferably glucose, in a biological medium, preferably in a flesh medium and more preferably in a flesh medium containing blood, having an accuracy which is very much greater than that of the devices and methods in the prior art.

### Brief description of the drawings

The characteristics of this invention may be understood more easily from the following brief descriptions of the drawings in which:
Figure 1 is an illustration of the electrical part of the device constructed according to the invention,
Figure 2 shows in sequential form the mathematical algorithm for calibrating measurements of analyte in the sample or biological medium in relation to the physiological characteristics of the skin of individual persons,
Figure 3 illustrates a practical embodiment of the device according to the invention, showing the sensors which analyse the physiological characteristics of an individual's skin and which produce values used to calibrate the glucose measurement,
Figure 4 illustrates a possible practical embodiment of a non-invasive glucose meter according to the invention.

### Detailed description of the invention

As mentioned previously, a first aspect of this invention relates to a device for the non-invasive measurement of glucose in a sample or biological medium. This sample or biological medium is preferably a flesh medium, and more preferably a flesh medium containing the individual's blood, such as the finger of a hand. Nevertheless the invention may also be used in non-fleshy areas or areas through which blood does not flow, such as interstitial fluid, adipose tissue, lymphatic fluid, etc. The patient's biological medium is placed in a receptacle and the optical detector receives light originating from the source which is attenuated by the biological medium. This measurement is corrected by the results of measurements of different physiological parameters of the skin produced by a series of specific sensors, such as elasticity, hydration, pH, temperature or melanin levels. These sensors are coupled to the glucose monitoring equipment which contains a mathematical algorithm which corrects the light values on the basis of the physiological characteristics of the individual, thus producing a measurement of glucose in biological media which is more accurate than in the case of the devices in the known art.

The second aspect of the invention relates to a method for measuring the parameter in a biological sample using near infrared spectroscopy which comprises the stages of measuring the said parameter by means of spectrophotometric techniques, preferably in the near infrared, and calibrating the values obtained through a mathematical algorithm which is capable of correcting the resulting values according the physiological characteristics of an individual person's skin.

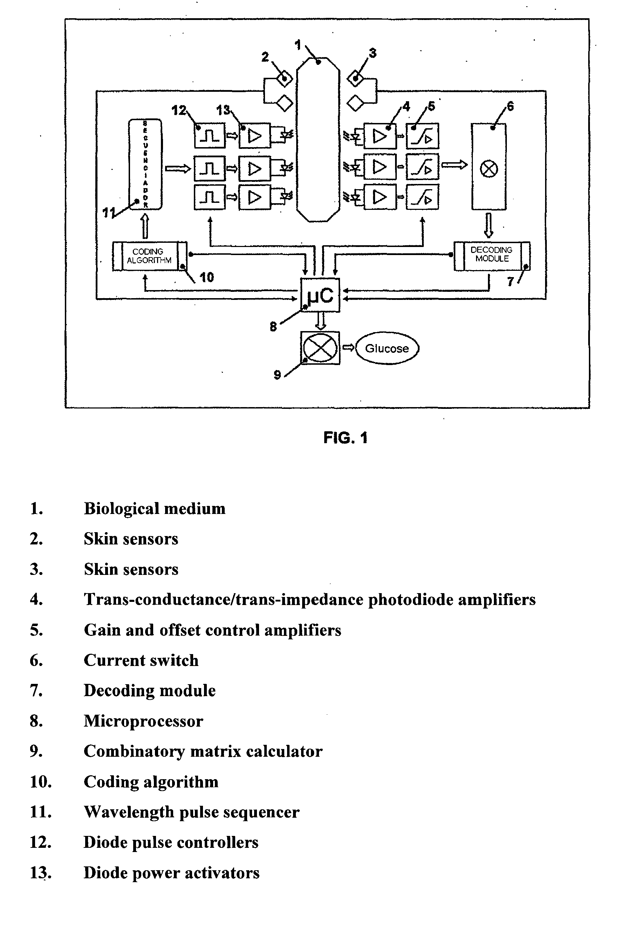

With reference to Figure 1, this shows diagrammatically the electronic part of the non-invasive device for monitoring glucose in a biological sample through a biological medium (1) such as the finger of a hand. The equipment comprises a microprocessor (8) which receives the parameters for the biological medium (1) in which measurement will be made through its sensors (2) and (3). Microprocessor (8) collects the best pulses of the light radiation and processes them using a coding algorithm (10) for the purpose. The processed pulse makes it possible to minimise interference between the different wavelengths, thus maximising the sensitivity of glucose detection. This processed pulse is delivered to a wavelength pulse sequencer (11) which by selectively activating the pulse controller (12) controls the specific width, height and frequency of the pulse required for detection. The resulting pulse is delivered to the power activators of diodes (13) of the LED type, which emit very precise quantities of light over known periods of time. This ray of light emitted by the LEDs passes through the biological medium (1) and is detected by a series of photodiodes. The small signal detected is amplified by trans-conductance amplifiers (4). The amplified signal obtained (offset) is adjusted in amplifiers (5) to find the best acquisition area for detection. Current switch (6) is used to select the wavelength's channel to decode and provide a representative signal of the medium analysed in a determined period of time. The other part of the coding algorithm (10) is the decoding module (7). The algorithm maximises the signal/noise ratio and shifts the interference between the channels. Finally, microprocessor (8) computes the glucose concentration in the medium through a matrix (9) using all the information received from the light and the sensor channels.

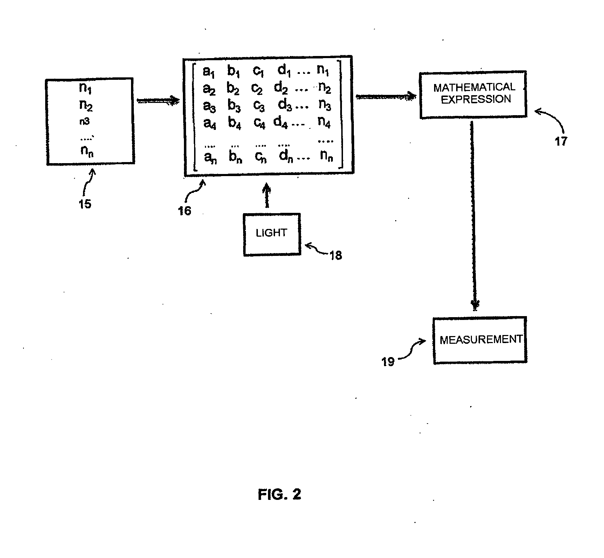

With reference to Figure 2, this illustrates a diagram of the mathematical algorithm used to correct the light values on the basis of the physiological parameters of an individual patient's skin produced by the corresponding sensors. Compartment (15) collects all the digital data obtained from the sensors analysing the skin, for example values for pH, pigmentation, blood pressure, oxygen saturation, pulse, skin moisture, fat, etc., among others. Compartment (18) generates values obtained by light passing through the skin incorporating a matrix of values in (16) which reflects the values obtained in compartments (15) and (18). Once the matrix has been generated, a mathematical expression or linearisation algorithm (17) which ultimately leads to the result for the glucose measurement (19) corrected by the physiological data for the individual's skin is obtained from this.

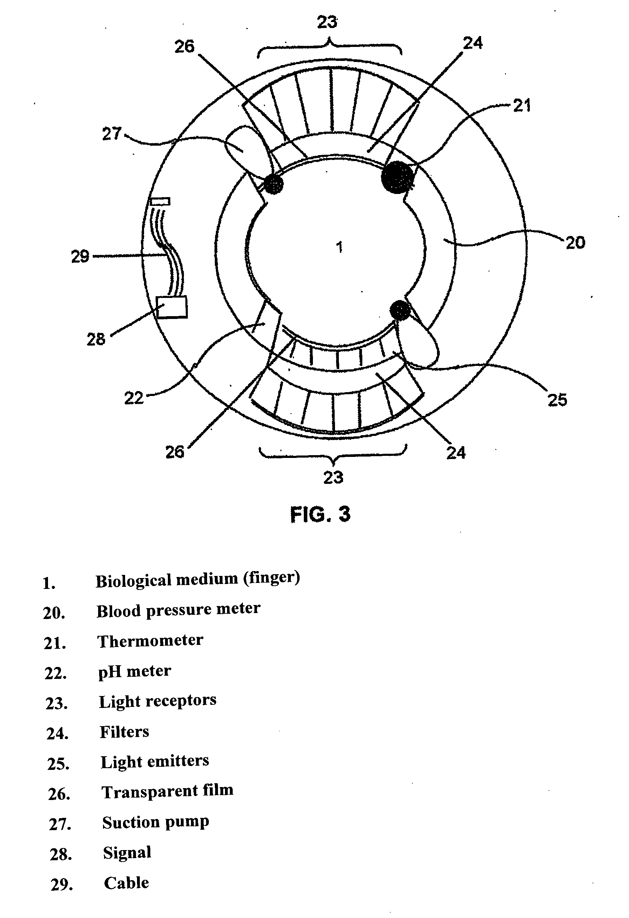

With reference to Figure 3, this illustrates a practical embodiment of the non-invasive device for measuring analytes in biological media through spectroscopy in the near infrared which is influenced by various parameters because it requires direct contact with an individual's skin. As previously discussed, a number of physiological factors of the skin such as melanin, elasticity and hydration of the skin, pH, temperature and blood pressure at the time when the glucose measurement is made in the sample, etc., have to be taken into account in order to increase the reliability of the measurement. In this figure the biological medium, for example a finger of an individual's hand, is represented by (1), and in order to determine the blood pressure a blood pressure meter or integrated pressure sensor (20) connected to an analogue signal conditioning circuit, a microcontroller and a group of displays is used to determine blood pressure. This design makes it possible to read the pressure in the sleeve and extract pulses to analyse and determine systolic and diastolic pressures and pulse per minute.

Body temperature is measured through thermometer (21). The pH value of the epidermis is in turn measured by pH meter (22). This datum is an important parameter for evaluating the quality of the hydrolipid layer of the skin.

Photodiodes or light receptors (23), for example of the PD23-02 type, are designed to detect radiation in the range 800-2300 nm. Preferably heterostructures with sensitive layers of InGaAsSb and AlGaAsSb windows are grown on GaSb substrates. These photodiodes are preferably manufactured in packets of 5.4 mm, have a photosensitive area having a diameter of 200 mm and have rapid response for use in the detection of high frequencies modulated by laser or emission LEDs.

Filters (24) are added preferably in an order such that the light is not significantly dispersed, and then again preferably a transparent film (26) is added to separate the parts of the finger and the apparatus.

Light emitting diodes (25), for example of the LED34 type, are designed to emit a spectral range around 3400 nm. Heterostructures (HS) are preferably grown on InAs substrates. Emitted outputs can be modulated by a forward flowing current. These diodes have been developed for use in optical gas sensors and medical diagnostics, have a stable power and a life of 10,000 hours. The wavelength used is 3.30-3.50 nm.

An absorption/reflection measurement is used to determine melanin in the skin. In order to do this light is emitted at preferably three specific wavelengths, and a receptor measures the light reflected by the skin. The positions of the emitter and receptor ensure that only diffuse and dispersed light is measured. When the quantity of emitted light is defined it is possible to calculate the quantity of light absorbed by the skin. Melanin is measured using specific wavelengths selected to correspond to different rates of absorption by pigments. Specific wavelengths are also used to measure erythema, and correspond to the maximum level of spectral absorption of haemoglobin, avoiding influence by other colours.

All the calibration data are located within the detector, so that the detector is wholly independent and can be connected to different types of sensors. The measurement is made using a detector comprising a single glass ring which contains all the elements of the sensor. The flat design of the head of the detector makes it possible to carry out direct measurements on the skin. Measurements are started by pressing a button located on a side of the detector body and the results appear immediately, showing, for example, up to two decimals. The short measurement time prevents occlusion effects on the skin. Likewise continuous measurements can be made.

Other useful accessories for the device according to the invention are the suction pump (27), the signal compartment (28) and the cable (29).

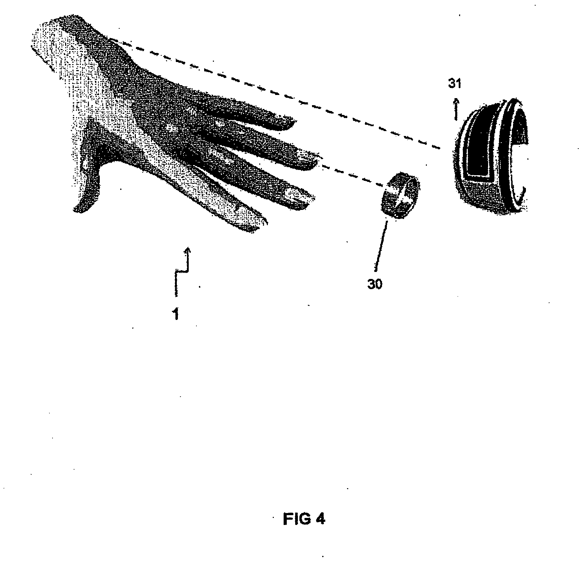

With reference to Figure 4, this illustrates a practical embodiment of the non-invasive device according to the invention for detecting levels of analytes and biological samples, such as glucose concentration levels in diabetic patients. Although this invention arises from the need to measure glucose concentration levels, it may also be applied to the detection of other blood components. Likewise, although the drawings have been produced with the aim of measuring glucose concentration in blood, the requirements discussed in this description apply directly to other sample options. In particular this process can in its broadest sense be applied to measurement of the concentration of any selected solute, including when all the major absorption peaks are located within a wavelength range in which this function is strongly absorbed by the solvent and/or other solutes. By "strongly absorbed" is meant that the absorption coefficient is greater than 10 cm⁻¹.

The device comprises a non-invasive device preferably in the form of a ring (30) connected to a miniature monitor which also preferably has the shape of a watch (31), which provides glucose measurements, preferably in real time. This device may optionally be connected to a remote medical centre or consulting doctor.

The sample is exposed to a light beam which excites the molecules of the solute into one or more excited states in which they emit light by the process of emission stimulated by infrared relaxation. One or more detectors designed to receive this part of the light emission produce an output signal which is indicative of the concentration of the solute selected. The intensity of the output signal from the detectors is affected by the following five factors: (i) fractional absorption of the beam before it reaches the selected solute, (ii) the absorption intensity of the solute selected for this beam, (iii) the emission intensity of the solute selected, produced per unit of flow of exposure of the beam to the solute molecules, (iv) the fraction of the light emission present which reaches the detector, and (v) the sensitivity of the detector for light of the wavelength selected.

The source of light, the exposure wavelength, the emitted wavelength, the path of the light through the solution, the path of the emitted light through the solution and the detector are selected to maximise the signal-noise ratio detected for the concentration of solute. A range of wavelengths is selected so that the photons have sufficient energy to excite the molecules of the solute to higher energy states, where a fall from which will be reflected by the emission of light at one or more wavelengths which are useful for quantifying the concentration of the solute.

In a preferred embodiment of the invention, one or more detectors for collecting the light emitted from the solute are located adjacent to the point from which the light is directed, thus substantially minimising the path of the emitted light from the exposed sample to the molecule detector. Preferably an emitted light wavelength is selected such that economic photodetectors for that wavelength are available on the market.

The incident beam is focussed on a small area around the source of that light so that the path through the solution is short and thus the solid angle generated by the detector for the light emitted from that region increases. This concentration of the beam may be achieved through the use of achromatic lenses, but in order to permit the incorporation of low-cost components it is preferable to use a source of monochromatic light so that achromatic aberrations do not significantly affect the signal/noise ratio of the output signal from the detector. Suitable sources include laser diodes and light emitting diodes (in particular light emitting diodes which produce a high light intensity at low cost). The sources of high intensity light (of the order of 5 watts/cm²) are useful for producing a sufficient quantity of light so that its absorption by the test solute and/or other solutes does not unduly degrade the amplitude of the detector signal. Preferably the intensity is at least 50 watts/cm , so that multiphoton processes can help excite the selected solute molecules.

The wavelength of the incident light is preferably in a range which enables the light to pass through the walls of the vessels containing the sample under test. In the case with which we are concerned, the scenario is a part of the patient's body and the sample is preferably one of the latter's fingers, as a result of which contact with the system analysing blood chemistry is easy. In this arrangement the wavelength of the radiation will be in the approximate range from 0.6 to 1.5 microns, given that this range of wavelengths is not strongly absorbed by the patient's epithelium. In addition to this, the skin is one of the driest parts of the human body, as a result of which absorption of the emitted light and exposure will be insignificant, as it passes through the epithelium. In a preferred embodiment of the invention, wavelengths selected from a range between 800 and 2500 nm, more preferably in at least one of the following wavelength ranges: 880-940 nm, 1190-1230 nm, 1600-1700 nm, 1730-1780 nm and 2300-2400 nm and more preferably of at least one of the following approximate wavelengths: 920 nm, 1210 nm, 1688 nm, 1750 nm and 2326 nm are used.

Because of the high absorption by water and other components of blood it is an advantage to expose a blood sample to an intense light beam, as most of the molecules in the selected component will pass into an excited state. By a "very intense beam" we mean a beam having an intensity of at least 5 watts/cm². Because of this high intensity it is an advantage that the molecular bonds should be relatively strong, so that the molecules of the components selected which are closest to the point at which the light beam is directed do not decompose in significant quantities. It is also preferable that this peak should not coincide with any absorption peak for other blood components, so that this incident energy is most efficient for the desired excitation.

The light which is emitted by the selected solute (blood glucose) and stimulates relaxation has peaks which are substantially similar, but have slightly greater wavelengths than the peaks in the absorption spectrum of the solute. In the case of concentration measurements for D-glucose anhydride (also known as "ADG", "blood glucose" or "blood sugar") there are five known absorption bands in this range. These bands are centred on the wavelengths 1040, 1085, 1109, 1160 and 1365 cm⁻¹. Unfortunately the following blood components can produce significant absorption in the following absorption bands - proteins at 1085 cm⁻¹, haemoglobin at 1109 cm⁻¹, urea at 1160 cm⁻¹ and all CH₂ groups at 1365 cm⁻¹. Only the band at 1040 cm⁻¹ does not coincide with any of these blood components which are active in the infrared. We therefore regard the wavelength of 1040 cm⁻¹ as the most preferable for measuring the concentration of D-glucose anhydride (ADG) molecules in blood.

The absorption band at 1040 cm⁻¹ corresponds to absorption by the C-O-C bond in the pyran ring of glucose in blood. This is fortunate, because this bond is particularly resistant to optical exposure to radiation in the visible or shorter wavelengths. The ADG molecule does not therefore easily break up into parts, even when strongly exposed to exciting light. This singular property is attributed to the strength of the C-O-C bond in the pyran ring in the molecular structure which enables it to be stretched, twisted or compacted by the exciting light without breaking up the molecule. When a molecule of ADG is bombarded by strong radiation to raise it from a base state to an excited state it is much more likely that the molecule will pass into an excited state through absorbing that radiation, rather than breaking up into parts. This excited molecule subsequently returns to its base state, through either elastic or inelastic processes. As a result of this characteristic the intensity of the light used to measure blood glucose concentration is greater than that which would normally be possible, which increases the sensitivity of this sensor of blood components.

These excited glucose molecules in blood can recover their base state through inelastic processes, such as collisions with other molecules or the walls of a confining structure. In this inelastic process the excitation energy is mainly converted into heat energy. These molecules can also recover their base state through an elastic process, normally known as stimulated emission relaxation, in which an excited molecule emits radiation at a wavelength which is characteristic for that molecule and at a wavelength which is slightly greater than that of the light which excited the molecule. This light change in wavelength occurs as a result of the conservation of energy and "moment" limitations in the transition. This incident light has a very narrow bandwidth, as is the case with laser light, and as a result of this we can concentrate on low cost optical systems in a very small region (around 50 microns diameter), only within the layer of the dermis and the upper part of the papillary zone which is closest to the epidermis. This location has been selected for focusing the beam because it is a region with a high concentration of blood and because it is not far (around 3 microns) from the epithelium. This being the case, the light produced and the emission relaxation of the excited glucose molecules only needs to travel a short focal distance from that region to a detector located in contact with the epidermis. Preferably the part of the epidermis through which this light is directed is in the front part of a finger (that is to say on the side opposite the nail), as this makes it easy for a diabetic to handle the system. Diabetics only need to place their fingers in the test apparatus in a place where a light beam can be "injected" through the epidermis into the papillary layer which is closest to that part of the epidermis.

The result of the measurement may be affected by the force with which a person places their finger in contact with the test equipment, because this can compress the blood at the finger, reducing the quantity of glucose in blood exposed to the light emission, with an apparent reduction in the blood glucose concentration. One or more additional sensors may be included in devices according to the invention to measure the light intensity, not only at 1040 cm⁻¹ , to take into account compensation for this change in pressure and the changes in radiation intensity which result when a person changes the temperature of the detection equipment. The wavelength of the light is therefore also selected to excite haemoglobin molecules (1109 cm⁻¹), and thus the light emitted by these molecules is of sufficient intensity to compensate for changes in blood concentration.

Given that the haemoglobin concentration in patients may vary, a calibration of the measurement is necessary to determine the patient's haemoglobin concentration for a known glucose level.

The incident light is collimated with a view to producing a pulsed output for the light emitted. This makes it possible to use synchronous detection to eliminate the approximately constant background from the emitted pulses which provide information about the blood component whose concentration is being measured.

A component concentration detector comprises a transparent plate on which a patient presses part of their epidermis, such as the front part of an index finger, in a test. One or more light beams are centered through this plate on the papillary part a close as possible to the epidermis to avoid any errors due to background radiation. A constant temperature of 37°C (normal body temperature) is maintained around this plate and the components so that the temperature does not change substantially when the patient presses his finger on the plate. As reference, in addition to selecting wavelengths, components which are characteristic of blood (e.g. haemoglobin) may be used to make the necessary corrections in calibrations to avoid the error produced by changes in finger pressure and the temperature of the plate as a result of contact with the finger of the patient's hand.

In a preferred embodiment of the invention the signal/noise ratio resulting from the measurement is processed using the technology known as orthogonal time-division multiplexing (OTDM). Some implementations of this, which may be used in the device and method according to the invention, are for example described in documents EP1311095 and EP1746759, where they are referred to as "GCM/OTDM". In these, by using a variety of modulations and advanced orthogonalisation techniques it is possible to obtain orthogonal channels from one SISO communication channel (single input/single output), providing a number of channels which are completely orthogonal in time. In this way a new channel which has MIMO behaviour is obtained. Unlike in OFDM, the information is distributed through orthogonal channels in time more than in some specific frequency bands. Through the optional use of this algorithm an amplification of the signal resulting from the measurement is obtained, and this substantially improves the sensitivity and accuracy of the blood glucose measurement produced by the device and method according to the invention.

## Claims

1. Device for the non-invasive monitoring of an analyte in a sample or biological medium (1) which comprises:
- at least one light emitter (25) which emits light radiation at a plurality of wavelengths selected in the near infrared (NIR),
- a receptacle for receiving the sample or biological medium (1) which is being monitored,
- at least one light receptor (23) positioned in such a way that it can receive the light radiation originating from the said light source (25) after passing through the said sample or biological medium (1) and which has been attenuated by the same, in which the said at least one light receptor (23) is capable of generating at least one output signal which is indicative of the intensity of the light radiation attenuated by the sample or biological medium (1), and
- a signal processor connected to the said light receptor (23) which is capable of receiving the at least one output signal emitted by the same and generating at least one signal indicative of the concentration of the analyte in the said sample or biological medium (1),
**characterised in that** the device also comprises:
- a plurality of sensors (20, 21, 22) for physiological parameters of the sample or biological medium (1) which generate values for each of the said parameters which are specific for the sample or biological medium (1) which is being monitored,
- a linearisation module which processes the values generated by the plurality of sensors (20, 21, 22) for physiological parameters of the sample or biological medium (1) and which produces a linearisation algorithm from those values, and
- a module for determining an analyte in the sample or biological medium (1) which on the basis of the at least one signal indicative of the concentration of analyte in the sample or biological medium (1) generated by the signal processor and the linearisation algorithm generated by the linearisation module determines the concentration of analyte in the sample or biological medium (1) corrected by the linearisation algorithm.

2. Device according to Claim 1, in which the plurality of wavelengths emitted by the at least one light emitter (25) is selected in a range of between 800 and 2500 nm.

3. Device according to Claim 2, in which the plurality of wavelengths emitted by the at least one light emitter (25) are selected from the group comprising at least one of the following wavelength ranges: 880-940 nm, 1190-1230 nm, 1600-1700 nm, 1730-1780 nm and 2300-2400 nm.

4. Device according to any one of above Claims 1-3, in which the plurality of sensors (20, 21, 22) for physiological parameters in the sample or biological medium (1) which give rise to specific parameters of the sample or biological medium (1) being monitored are selected from the group comprising sensors for blood pressure, temperature, pH, skin pigmentation, oxygen saturation, pulse, skin humidity and fat content, alone or in any combination thereof.

5. Device according to any one of above Claims 1-4, in which the linearisation module processes the values generated by the plurality of sensors for physiological parameters in the sample or biological medium (1) in a data matrix (16) which through a mathematical expression (17) generates the algorithm for linearising the signals generated by the signal processor.

6. Device according to any one of Claims 1-5, which also comprises a signal amplification device which reduces noise and minimises interference to maximise the signal.

7. Device according to Claim 6, in which the signal amplification device comprises electronic equipment making use of a coder/decoder algorithm to minimise interference between light beams of different wavelengths and to maximise the signal/noise ratio and detection sensitivity.

8. Device according to Claim 7, in which the coding/decoding algorithm is based on orthogonal time division multiplexing (OTDM).

9. Device according to Claim 6, in which the amplification device comprises electronic equipment which takes into account the influence of the environmental parameters of the measurement medium and uses them to maximise spectroscopic efficiency through computing light pulses at different wavelengths.

10. Device according to any one of the preceding claims, in which the analyte is selected from the group comprising glucose, haemoglobin, oxygen, cholesterol, uric acid and lactic acid.

11. Non-invasive method for measuring an analyte in a sample or biological medium (1) using near infrared spectroscopy (NIR) comprising the stages of:
- illuminating the sample or biological medium (1) with light radiation at a plurality of wavelengths selected in the near infrared (NIR),
- detecting the said light after it has been attenuated by the sample or biological medium (1),
- generating at least one output signal indicative of the intensity of the light radiating attenuated by the sample or biological medium (1) from the said attenuated light, and
- generating at least one signal indicative of the concentration of the analyte in the sample or biological medium (1) on the basis of the at least one output signal indicative of the intensity of the light radiation attenuated by the sample or biological medium (1),
**characterised in that** the method further comprises the stages of:
- measuring a plurality of physiological parameters in the sample or biological medium (1), generating values for each of the said physiological parameters which are specific to the sample or biological medium (1) which is being monitored,
- generating a linearisation algorithm from the values of each of the physiological parameters which are specific to the sample or biological medium (1) which is being monitored, and which produces a linearisation algorithm from those values, and
- determining the concentration of the analyte in the sample or biological medium (1) corrected by the linearisation algorithm from the at least one signal indicative of the concentration of the analyte in the sample or biological medium (1 ) and the linearisation algorithm.

12. Method according to Claim 11 above, in which the analyte is selected from the group comprising glucose, haemoglobin, oxygen, cholesterol, uric acid and lactic acid.

13. Method according to any one of above Claims 11-12, in which the physiological parameters which are specific to the sample or biological medium (1) which is being monitored are selected from the group comprising blood pressure, temperature, pH, skin pigmentation, oxygen saturation, pulse, skin humidity and fat content, alone or in any combination thereof.

14. Method according to any one of above Claims 11-13, in which the method comprises the additional stage of applying coding/decoding algorithm to minimise interference between light beams of different wavelengths and to maximise the signal/noise ratio and detection sensitivity.

15. Method according to Claim 14 above, in which the said algorithm is based on orthogonal time division multiplexing (OTDM).
